Europäisches Patentamt

European Patent Office

Office européen des brevets

(19)

(11) Publication number: **0 069 322**
**B1**

(12) # EUROPEAN PATENT SPECIFICATION

(45) Date of publication of patent specification: 24.08.88

(21) Application number: 82105731.2

(22) Date of filing: 28.06.82

(51) Int. Cl.⁴: **B 01 J 27/18, C 07 C 85/06,**
**C 07 D 295/00, C 07 D 487/08**
**// (C07D487/08, 241:00,**
**241:00)**

(54) **Catalysis of condensation reactions of amines.**

(30) Priority: 29.06.81 US 278814
24.05.82 US 381232
24.05.82 US 381233

(43) Date of publication of application:
12.01.83 Bulletin 83/02

(45) Publication of the grant of the patent:
24.08.88 Bulletin 88/34

(84) Designated Contracting States:
AT BE CH DE FR GB IT LI LU NL SE

(56) References cited:
GB-A- 904 182
US-A-2 985 658
US-A-3 297 701
US-A-3 507 836
US-A-3 933 932
US-A-3 957 900
US-A-4 250 343

(73) Proprietor: AIR PRODUCTS AND CHEMICALS,
INC.
P.O. Box 538
Allentown, Pennsylvania 18105 (US)

(72) Inventor: Wells, James Edward
2115 Bryn Mawr Place
Ardmore, PA 19003 (US)
Inventor: Eskinazi, Victoria
3360 Chichester Avenue Apartment P-11
Boothwyn, PA 19061 (US)

(74) Representative: Sandmair, Kurt, Dr. et al
Patentanwälte Schwabe, Sandmair, Marx
Stuntzstrasse 16
D-8000 München 80 (DE)

## Description

Technical field of the invention

The present invention relates to organic condensation reactions effected in the presence of phosphate catalysts and is more particularly concerned with the production of amine compounds in enhanced yields.

Background of the prior art

Organic synthesis by condensation reactions resulting in the loss of a molecule of water or of ammonia are well known in the art. Certain of such reactions are generally effected in the presence of acidic catalysts. An important area in which such acid-catalysis has been employed is in cyclization reactions as in the synthesis of triethylenediamine and its C-substituted homologues. The catalysts more generally used or proposed for use in such cyclization reactions are solid products of the Lewis acid type.

Triethylenediamine, also called diazabicyclo-[2.2.2]-octane, has been widely employed commercially as a catalyst in organic isocyanate reactions with compounds containing labile hydrogen, as in the production of urethane polymers. Triethylenediamine (sometimes hereinafter referred to as TEDA) was initially prepared in significant quantities by methods such as that described in U.S. Patent No. 2,937,176, by passing aliphatic amines in vapor phase over acidic cracking catalyst, such as silica-alumina dried gel or acid-activated clays. Numerous other feed stocks as well as other catalysts are disclosed in subsequent patents for preparation of TEDA as well as C-alkyl derivatives thereof.

Typical among these are U.S. Patents 2,985,658 and 3,166,558 employing preferably silica-alumina type catalyst, but listing also other useful solid acid catalysts that can be employed such as alumina in which phosphate or fluoride ion is incorporated (U.S. 2,985,658).

Among other catalysts proposed in the patent art for preparation of triethylene diamine and/or C-alkyl homologues thereof, are certain phosphate compounds, particularly aluminum phosphate.

The use of aluminum phosphate as a catalyst in the preparation of heterocyclic compounds from aliphatic amines were early disclosed in U.S. Patent 2,467,205, particularly for the preparation of piperazine from ethylenediamine or from polyethylene polyamine. The use of aluminum phosphate as catalyst in the preparation of triethylenediamine accompanied by piperazine among other by-products is further described in U.S. Patent 3,172,891; while U.S. Patent 3,342,820 describes the use of complex phosphates of alkali metal and trivalent metals in the preparation of C-alkyl TEDA.

U.S. Patent 3,297,701 discloses as catalysts for preparation of TEDA and C-alkyl TEDA, in addition to the preferred aluminum phosphate stated to be superior, other phosphate compounds including calcium and iron phosphates among other listed metal phosphates. In the conversion of N-aminoethylpiperazine to triethylenediamine over aluminum phosphate catalyst, at most up to 39 mol% triethylenediamine is said to be obtained. Other of the named metal phosphate catalysts in the examples of the patent obtain yields of less than 10 mol% TEDA.

Acid metal phosphate catalysts, particularly phosphates of boron, aluminum and trivalent iron, have also been proposed for use in intramolecular cyclic dehydration reactions and other condensation reactions involving amino compounds. Examples of such reactions are found in U.S. Patent 4,117,227, which discloses conversion of an N-substituted diethanolamine to the corresponding N-substituted morpholine. U.S. Patent 4,036,881 describes preparation of non-cyclic polyalkylene polyamines by condensation of an alkylene diamine with an ethanolamine. N-hydroxyethylmorpholine is condensed with morpholine in the presence of aluminium phosphate catalyst to form dimorpholino ethane according to U.S. Patent 4,103,087. Similarly, dimorpholinodiethyl ether is obtained by condensation of hydroxyethyl morpholine with aminoethyl morpholine over iron, aluminum or boron phosphate in U.S. Patent 4,095,022. Reaction of piperazine with ethanolamine over such acidic metal phosphate produces N-aminoethyl piperazine according to U.S. Patent 4,049,657. U.K. Patent 1,492,359 discloses the preparation of morpholine compounds by reacting an aminoalkoxyalkanol compound over phosphoric acid and similar types of phosphorus-containing substances.

Pyrophosphates of lithium, sodium, strontium and barium have been used as dehydration catalysts; see U.S. Patent 3,957,900. Phosphates and pyrophosphates of strontium and nickel have been used for the dehydrogenation of, for example, n-butene to butadiene under the conditions described in U.S. Patent 3,541,172.

Summary of the invention

It has now been found that high yields of aliphatic alkylamines by condensation reaction of lower aliphatic alkylamines or ammonia with an alcohol are obtained by carrying out the reaction in the presence of monohydrogenphosphate of strontium or lanthanum, or of triethylene diamine by converting a substituted piperazine in the presence of monohydrogenphosphate of strontium.

Detailed description of the invention

The monohydrogen and dihydrogen phosphate catalysts of the present invention are prepared by reaction of a monohydrogenphosphate of an alkali metal or ammonium with a soluble salt of strontium or lanthanum at ambient temperatures. The highest purity and best yields of the present invention are obtained when using the soluble metal salts of a strong acid such as the metal nitrates, in substantially stoichiometric proportion to the phosphate. In aqueous media under these conditions, the reaction mixture is at a pH of about 3.5 to 6.5. In general, to obtain a precipitate of desired high content of the metal monohydrogen phosphate, the ratio of phosphate to metal salt in the reaction mixture should be such as to have a pH of 5±3, or the mixture should be adjusted to that pH range.

For use as a catalyst, the metal monohydrogen phosphate product may be employed in the form of irregular particles of the desired size range prepared by breaking up the washed and dried filter cake or in the form of regular shaped pellets obtained by known methods of casting or extruding or the product may be deposited or otherwise impregnated into the pores of a microporous substrate such as alumina, silica, silica-alumina, and the like. In using the catalyst of the present invention to catalyze the condensation reactions, substantially the same conditions may be employed as when using the known catalysts for the particular synthesis. For optimum results, however, some adjustment in temperature, diluent and/or space rate may be found beneficial.

Some specific examples of the type of organic compounds selectively obtained by the method of this invention include TEDA, and the aliphatic alkylamines such as methylamine, methylethylamine, dimethylethylamine, and dimethylaminoethylmorpholine. In the production of these compounds, the temperature is in the range of about 285° to 420°C, the pressure is in the range of about 0.1 to 1.5 atmospheres, and the liquid hourly space velocity (LHSV) of the organic feed stock per volume of catalyst is in the range of about 0.05 to 1.5. Preferably depending on the particular reaction, the temperature is in the range of about 300° to 400°C, the pressure is in the range of about 0.3 to 1.0 atmospheres and the LHSV is in the range of about 0.1 to 0.3 to obtain the highest yields and most economical process. The operable ratio of the organic feeds to water diluent is about 10 to 90% on a weight basis and preferably, 20 to 80% by weight. The optimum yield of these compounds is likely to be obtained using the highest temperature in the preferred range at the lowest LHSV.

In the preparation of TEDA, the organic feed stock used is a substituted piperazine compound selected from the group consisting of hydroxyethylpiperazine and aminoethylpiperazine. The catalysts of this invention are relatively unaffected by the purity of the feed stock. For example, high conversion and good yields can be obtained from crude hydroxyethylpiperazine which contains minor quantities of piperazine and bis hydroxethylpiperazine.

The methods and catalysts of this invention are also capable of reacting an alcohol and a nitrogen-containing compound selected from the group consisting of ammonia or aliphatic primary and secondary amines, to selectively convert this compound to the corresponding symmetrical or unsymmetrical amine with little, if any, conversion to the corresponding by-products of thermodynamic amine equilibration. The amines and alcohol in the feed stock each contain 1 to 20 carbons per molecule. Preferably, the catalyst is lanthanum monohydrogen phosphate and the molar ratio of alcohol to nitrogen-containing compound ranges from about 1 to 6 to 6 to 1.

Catalyst preparation
Example 1

200 grams of strontium nitrate [$Sr(NO_3)_2$] was dissolved in distilled water and brought to a total volume of 800 cc with distilled water. To this solution there was added 10 cc of 85% phosphoric acid followed by 34.5 cc of 50% sodium hydroxide added rapidly with vigorous stirring. The resultant fine white precipitate was stirred for 10 minutes, vacuum-filtered and water-washed. The obtained filter cake was air dried in a static oven at approximately 110°C and extruded into 1/8 inch pellets for evaluation.

The obtained product had a surface area of 10—15 m²/g. By X-ray diffraction the principal component was identified as $\beta$-$SrHPO_4$ with minor quantities of $Sr_5(OH)(PO_4)_3$ and unreacted $Sr(NO_3)_2$. Infrared spectroscopy showed a spectrum consistent with $SrHPO_4$. (Ref: Richard A. Nygurst and Ronald O. Kagel, "Infrared Spectra of Inorganic Compounds", page 163, 1971).

Example 2

212 grams of $Sr(NO_3)_2$ were dissolved in distilled water and diluted to 500 cc. 132 grams of dibasic ammonium phosphate —$(NH_4)_2HPO_4$— were dissolved in distilled water and diluted to 500 cc with heat. The three salt solutions were then combined with heat and stirred for about 10 minutes. The combined solution was vacuum filtered and the resulting precipitate was washed with distilled water and air dried overnight in a static oven at approximately 110°C. The filter cake was broken up into small (0.32—0.64 cm; 1/8 to 1/4 inch) irregular granules for evaluation. The resulting product had a surface pH of 4,8—5,2 as determined by acid base indicators.

Examples 3—4

The following salts were combined and catalysts were prepared in the manner set forth under Example 2:

| Example | Salt solutions (a) | (b) | Catalyst formulation | Surface pH |
|---|---|---|---|---|
| 3 | 415 g. La(NO$_3$)$_3$ · 5H$_2$O | 198 g. (NH$_4$)$_2$HPO$_4$ | La$_2$(HPO$_4$)$_3$ | 0.2—1.8 |
| 4 | 202 g. Sr(NO$_3$)$_2$+ 20 grams of La(NO$_3$)$_3$ · 5H$_2$O | 132 g. (NH$_4$)$_2$HPO$_4$ | SrHPO$_4$/LaHPO$_4$ (Sr/La=14.9/l) | 4—5 |

Controls 1—3

The following salts were also combined in the manner of the Example 1 preparation.

| Control | Salt solutions (a) | (b) | Catalyst formulation |
|---|---|---|---|
| 1 | 261 g. Ba(NO$_3$)$_2$ | 230 g. NH$_4$HSO$_4$ | BaSO$_4$ |
| 2 | 75 g. CsCl | 40 g. (NH$_4$)$_2$HPO$_4$ | CsHPO$_4$* |
| 3 | 106 g. Sr(NO$_3$)$_2$ | 40 g. 50% NaOH+ 80 g. (NH$_4$)$_2$H$_2$AsO$_4$ | SrHAsO$_4$ |

*Did not form a precipitate.

Control 4

200 grams of Sr(NO$_3$)$_2$ were dissolved in distilled water and diluted to 400 cc. 92 grams of H$_2$SO$_4$ were diluted in 200 cc. of distilled H$_2$O. 75 grams of 50 wt.% NaOH solution were diluted to 200 cc. with distilled water. The H$_2$SO$_4$ and NaOH solutions were mixed together slowly. The Sr(NO$_3$)$_2$ solution was stirred into the solution containing H$_2$SO$_4$ and NaOH. The solution was stirred for 10 minutes and the precipitate was filtered, washed and dried. The surface pH of the resulting catalyst was less than 3 which was believed to be substantially all SrSO$_4$.

Example 5

2000 grams of Sr(NO$_3$)$_2$ was dissolved in 2000 cc of deionized water and the solution diluted to 4000 cc with deionized water after dissolution of the Sr(NO$_3$)$_2$ was complete.

In another container, 1342.3 grams of Na$_2$HPO$_4$ were dissolved in 2000 cc of deionized water. After solution of the Na$_2$HPO$_4$ was complete, the solution was diluted to 4000 cc with deionized water. The pH of this solution was approximately 8.8.

Precipitation of SrHPO$_4$ was effected by slowly adding the Na$_2$HPO$_4$ solution to the Sr(NO$_3$)$_2$ solution with rapid stirring. The white SrHPO$_4$ precipitated rapidly from solution forming a rather thick slurry. This slurry was mixed for one hour, after which time the pH was measured to be about six.

The solid SrHPO$_4$ was recovered by filtering on an eight frame filter press using cloth filters. It was washed with deionized water. After filtering and washing, the solid was dried in a circulating hot air oven at 121°C (250°F) for four hours. The yield of SrHPO$_4$ was 1680 grams. The solid was wetted and formed into pellets by extrusion through a 3.1 mm die plate and cutting the extrudates to about 1/4 inch in length. After drying the extrudate at 121°C (250°F) for fours in a circulating hot air oven, they were heat treated at 350°C (662°F) for two hours in a 20% steam, 80% air atmosphere.

Use of catalysts

Examples 6—14

Each of the products prepared in accordance with Examples 1 through 5 and Controls 1 and 3—5 above were evaluated for catalytic performance for the preparation of TEDA with either a feed mixture containing hydroxyethylpiperazine (HEP) or N-aminoethylpiperazine (AEP) in accordance with the following test procedure:

(a) 20 cc (approximately 6.2 g.) of the catalyst was loaded into a 1,9 cm (3/4") diameter stainless steel reactor.

(b) The reactor was placed in a conventional tube furnace such that the catalyst bed was near the furnace center and therefore could be heated to a constant and uniform temperature.

(c) The catalyst bed temperature was raised to a temperature of 340°—400°C over a period of 15 to 30 minutes while a small flow of gaseous nitrogen was maintained through the reactor to aid in the removal of water vapor.

(d) A feed mixture containing HEP and water such that the organic component made up 60% of the mixture was then allowed to flow through the catalyst bed at a rate of 6.5—7.0 cc/hour; the nitrogen flow was discontinued.

(e) The catalyst bed temperature indicated in the tables set forth below was maintained during the run and the product samples were collected and analyzed. Analyses were performed using well-established gas chromatographic techniques.

The yields of TEDA and piperazine (PIP) as well as the conversion obtained from the catalysts of Examples 1—4 in Table 1 can be compared with those of Control Catalysts 1 and 3—5 in Table 2 below.

TABLE 1
TEDA production

| Example | Cat. of Ex. | Form | Nominal formulation | TEDA yield, Wt. % | PIP yield, Wt. % | Conversion mol. % | Temp., °C | Feed |
|---------|-------------|------|---------------------|-------------------|------------------|-------------------|-----------|------|
| 6 | 1[(1)] | Granules | $SrHPO_4$ | 71.5 | 3.7 | 99.3 | 360 | HEP |
| 7 | 1[(1)] | Granules | $SrHPO_4$ | 80.7 | 3.4 | 99.3 | 360 | HEP |
| 8 | 1 | Granules | $SrHPO_4$ | 71.1 | 7.6 | 95.4 | 370 | HEP |
| 9 | 2 | Granules | $SrHPO_4$[(4)] | 76.0 | 7.8 | 98.6 | 360 | HEP |
| 10 | 2 | Granules | $SrHPO_4$ | 29.0 | 21.0 | 99.0 | 400 | AEP |
| 11 | 3 | Granules | $La_2(HPO_4)_3$ | 16.2 | 4.2 | 98.8 | 340 | HEP |
| 12 | 3 | Granules | $La_2(HPO_4)_3$ | 25.5 | 3.3 | 95.2 | 380 | AEP |
| 13 | 4 | Granules | $La/SrHPO_4$ (La/Sr=1/15) | 58.7 | 5.3 | 99.2 | 360 | HEP |
| 14 | 4 | Granules | $La/SrHPO_4$ (La/Sr=1/15) | 27.4 | 14.6 | 96.1 | 380 | AEP |

[(1)]Based on average of two duplicate runs.
[(4)]Analysis of catalyst after reaction to produce TEDA indicates the formation of a minor amount of $SrP_2O_7$.

TABLE 2

| Catalyst of control | Form | Nominal formulation | TEDA yield, Wt. % | PIP yield, Wt. % | Conversion mol. % | Temp., °C | Feed |
|---------------------|------|---------------------|-------------------|------------------|-------------------|-----------|------|
| 1 | Granules | $BaSO_4$ | 14.3 | 2.9 | 31.2 | 360 | HEP |
| 3 | 25% coated spheres | $SrHA_sO_4$ | 4.6 | 6.2 | 36.2 | 340 | HEP |
| 3 | 25% coated spheres | Sr⊦ $SrHA_sO_4$ | 1.2 | 1.6 | 43.5 | 360 | AEP |
| 4 | Granules | $SrSO_4$ | 2.1 | 27.3 | 36.4 | 360 | HEP |
| 5[(6)] | Granules | $AlPO_4$ | 28.4 | 6.0 | 100 wt.% | 400 | HEP |
| 5[(7)] | Granules | $AlPO_4$ | 33.9 | 26.7 | 83 wt.% | 375 | AEP |

[(6)]Data obtained from Example II of U.S. Patent 3,297,701.
[(7)]Data obtained from Example XXI of U.S. Patent 3,297,701.

The results set forth in Table 1 where the catalysts of the examples demonstrate a unique ability to convert over 50 mol.% of the feed to products the majority of which is TEDA.

Example 15
The catalyst of Example 5 was tested in the conversion of crude HEP to TEDA. The reaction was carried out at atmospheric pressure, at a liquid hourly space velocity of 0.3 and at the temperatures indicated in Table 3 below.

5

TABLE 3

| | Initial | After 78 days |
|---|---|---|
| Bed temp., °C | 360 | 368 |
| HEP conversion, wt.% | 99+ | 99+ |
| TEDA yield, wt.% | 40.5 | 43.0 |
| PIP yield, wt.% | 13.5 | 18.5 |

Example 16

The catalyst of Example 5 was tested for the conversion of diethanolamine to TEDA. The test was carried out at 370°C using a feed consisting of diethanolamine and water (2.0 to 1.0 mole ratio) pumped into the reactor at a rate of 4.4 liquid cc/hr along with helium diluent at a rate of 25 cc/minute. The diethanolamine was in completely converted to TEDA, as the only, i.e. about 26 mol.%, recovered product.

Example 17

A 64% by weight solution of N-aminoethyl piperazine in water was passed over a catalyst composition consisting essentially of $SrHPO_4$ at 380°C and at a liquid hourly space velocity of 0.3 volumes of liquid per volume of catalyst. In a first pass operation there was obtained 96.8 mol.% conversion of the feed compound, obtaining a yield of 34.8% by weight (40.1 mol.%) TEDA and 27.1% by weight (40.6 mol.%) PIP.

Other typical condensation reactions in which $SrHPO_4$ may be employed as a catalyst include the formation of amines by amination of the corresponding alcohols with ammonia and the formation of polyamines from glycols and diamines.

It is believed that the key to the properties of the $SrHPO_4$ as a highly selective catalyst is due to the presence of a specific structure, which provides a narrow range of acidity. This narrow acidity range displayed by $SrHPO_4$ may be optimum for promoting certain types of acid catalyzed reactions, in contrast to such catalysts as alumina, silica-alumina and the like which have acid sites of widely varying strength, and hence show relatively low selectivity for the desired reaction.

Examples 18—21

The performance of the catalyst was evaluated for the preparation of an aliphatic secondary amine with a feed mixture of diethylamine (DEA) or monoethylamine (EA) and methanol using the general procedure used in Examples 6—14 for the preparation of TEDA. Specifically, 1 mol. of the primary amine and 1 mol. of the alcohol were reacted at 350°C, 1 atmosphere pressure and an LHSV of 0.15/hr.

3 grams of the $La_2(HPO_4)_3$ catalyst of Example 3 were coated onto alumina spheres. The results from these reactions are summarized in Table 4 below.

Examples 18 and 19

The $SrHPO_4$ catalyst of Example 2 was used to convert methanol and either monoethylamine (Example 20) or diethylamine (Example 21).

The results of Examples 18—21 are summarized in Table 4 below.

TABLE 4

Aliphatic amine production

| Example | Amine feed | Yield, mol. % | | | Selectivity, mol.% | | | Conversion Mol.% |
|---|---|---|---|---|---|---|---|---|
| | | MEA | DMEA[7] | DEMA | MEA | DMEA | DEMA | |
| 18 | EA | 27 | 25 | — | 47 | 44 | — | 57 |
| 19 | DEA | — | — | 39 | — | — | 66 | 59 |
| 20 | EA | 22 | 17 | — | 55 | 17 | — | 40 |
| 21 | DEA | 1.4 | 6.9 | 41 | 2.8 | 13.8 | 82 | 50 |

[7]Dimethylethylamine

The data of Table 4 above illustrates the unexpectedly high selectivies to the corresponding aliphatic amine without the formation of the corresponding byproducts of thermodynamic amine equilibration. The relatively low conversion to product of Examples 18—19 can be attributed to the fact that only 3 gms. $La_2(HPO_4)_3$ were used in Examples 18—19 in comparison to about 20 gms. of $SrHPO_4$ that were used in Examples 20 and 21.

Example 22

The $La_2(HPO_4)_3$ catalyst of Example 3 was deposited on alumina spheres and the procedure of Example 18 was followed except that 1 mol of ammonia was substituted for 1 mole of EA. The results were yields based on the methanol in the feed of 13 mol.% trimethylamine (TMA), 2.8 mol.% dimethylamine (DMA) and 3 mol.% monomethylamine (MA), selectivities based on the methanol of 69 mol.% TMA, 14.9 mol.% DMA and 16 mol.% MA, and a methanol conversion of 52 mol.%.

Examples 23—26

The $SrHPO_4$ catalyst of Example 2 was evaluated for the preparation of N-(2-dimethylaminoethyl)-morpholine (DMAEM) with a feed mixture of morpholine (MOR), dimethylethanolamine (DMEA), distilled water, hydrogen and helium in the amounts shown below in Table 5 using the general procedure used in Examples 6—14 for the TEDA preparation. Specifically the condensation reaction was carried out at 340°C, 1 atmosphere of pressure and an LHSV in the range of 0.31—0.44/hr. as shown in Table 5 in the presence of 20 cc. of catalyst granules.

The results of these runs are summarized in Table 5 below.

TABLE 5
DMAEM production

| Example | Feed, Vol.% | LHSV, hr.$^{-1}$ | Yield, Mol.% MOR | Conversion, Mol.% |
|---|---|---|---|---|
| 23 | MOR 40<br>DMEA 20<br>$H_2O$ 20<br>He (20 cc/min) | 0.21 | 42 | 32 |
| 24 | MOR 40<br>DMEA 40<br>$H_2O$ 20<br>He (20 cc/min) | 0.21 | 37 | 28 |
| 25 | MOR 60<br>DMEA 20<br>$H_2O$ 20<br>He (20 cc/min) | 0.21 | 43 | 22 |
| 26 | MOR 20<br>DMEA 60<br>$H_2O$ 20<br>He (20 cc/min) | 0.21 | 33 | 45 |

## Claims

1. A process for the synthesis of aliphatic alkylamines by condensation reactions of lower aliphatic alkylamine or ammonia with an alcohol in the presence of phosphate catalysts, characterized in that the reaction is carried out in the presence of monohydrogen phosphate of strontium or lanthanum.

2. A process for the synthesis of triethylene diamine by converting a substituted piperazine characterized in that the reaction is carried out in the presence of monohydrogen phosphate of strontium.

## Patentansprüche

1. Verfahren zur Synthese von aliphatischen Alkylaminen durch Kondensationsreaktionen von niedrig-aliphatischem Alkylamin oder Ammoniak mit einem Alkohol in Gegenwart von Phosphatkatalysatoren, dadurch gekennzeichnet, daß die Reaktion in Gegenwart von Strontium- oder Lanthan-monohydrogenphosphat durchgeführt wird.

2. Verfharen zur Synthese von Triethylendiamin durch Überführung eines substituierten Piperazins, dadurch gekennzeichnet, daß die Reaktion in Gegenwart von Strontium-monohydrogenphosphat durchgeführt wird.

## Revendications

1. Procédé pour la synthèse d'alkylamines aliphatiques par des réactions de condensation d'alkylamine aliphatique inférieure ou d'ammoniaque avec un alcool en présence de catalyseurs consistant en phosphates, caractérisé en ce que la réaction est effectuée en présence de phosphate monoacide de strontium ou de lanthane.

2. Procédé pour la synthèse de triéthylène diamine par conversion d'une pipérazine substituée, caractérisé en ce que la réaction est effectuée en présence de phosphate monoacide de strontium.